# EUROPÄISCHE PATENTANMELDUNG

(11) **EP 2 316 342 A1**
(43) Veröffentlichungstag der Anmeldung: **04.05.2011**
(21) Anmeldenummer: 09405160.4
(22) Anmeldetag: 18.09.2009
(51) Int. Cl.: A61B 6/03, F16C 19/50, F16C 29/04

(54) **Trägersystem für Scannergeräte**

(71) Anmelder: Güdel Group AG, 4900 Langenthal (CH)
(72) Erfinder: Roth, Ernst, 4900 Langenthal (CH); Zulauf, Walter, 4937 Ursenbach (CH)
(74) Vertreter: Rüfenacht, Philipp Michael

(57) **Zusammenfassung**

Die Erfindung betrifft ein Trägersystem für ein Scannergerät, welches einen Rahmen (1) und eine Trommel (2) umfasst. Die Trommel (2) besteht im Wesentlichen aus vergütetem Stahl und ist durch ein Laufrollensystem am Rahmen (1) gelagert. Dieses Laufrollensystem besteht aus Laufrollen (4,5), welche aus gehärtetem Stahl bestehen oder mindestens mit Laufflächen aus gehärtetem Stahl bestückt sind. Die Laufrollen (4,5) sind am Rahmen (1) befestigt und erlauben eine Drehung der Trommel (2) um die rotationssymmetrische Achse der Trommel (2). Für ein Scannergerät sind eine Strahlungsquelle und ein oder mehrere Detektoren an der Trommel (2) befestigbar.
Durch die Herstellung der Trommel aus vergütetem Stahl und durch die Laufrollen, welche zumindest Laufflächen aus gehärtetem Stahl aufweisen, können erhöhte Drehgeschwindigkeiten der Trommel erreicht werden, ohne dass zugleich vermehrt Vibrationen auftreten. Die so erreichten Drehgeschwindigkeiten ermöglichen kurze Untersuchungszeiten des Scannergerätes, da vibrationsbedingte Untergrundsignale vermieden werden. Ausserdem wird eine unvorteilhafte Geräuschentwicklung während der Untersuchung vermieden, welche den Komfort für die Nutzer des Scannergerätes verringern würde.
Somit eignet sich das erfindungsgemässe Trägersystem für Scannergeräte besonders für Anwendungen, bei welchen kostengünstig eine kurze Untersuchungszeit erwünscht ist.

## Beschreibung

### Technisches Gebiet

Die Erfindung betrifft ein Trägersystem für ein Scannergerät, welches einen Rahmen und eine Trommel umfasst, wobei eine Lagerung der Trommel am Rahmen eine Rotation der Trommel um deren rotationssymmetrische Achse erlaubt und wobei an der Trommel ein Scannersystem befestigbar ist.

### Stand der Technik

Trägersysteme für Scannergeräte der oben genannten Art sind bekannt. Beim Scannersystem kann es sich insbesondere um eine Röntgen-Strahlungsquelle und einen oder mehrere entsprechende Detektoren handeln. Die DE 196 29 931 C2 (Siemens) beschreibt beispielsweise einen Röntgen-Computertomographen mit einer ringförmigen Gantry, welche drehbar in einem Rahmen gelagert ist, der Rollen für die radiale und axiale Führung der Gantry aufweist. Die Rollen sind mit ihren Achsen um 90° gegeneinander versetzt und laufen auf Aussen- bzw. Seitenflächen von Schienen, die die Gantry an ihrem Umfang umschliessen. Die Schienen sind unter Zwischenschaltung einer Gummischicht mit der Gantry verbunden.

Die DE 195 81 512 C2 (Analogic) betrifft ein Röntgenstrahl-Tomographie-System mit einem schwenkbaren Rahmen, an welchem eine drehbare Trommel angeordnet ist. Auf der Trommel sind Funktionselemente des Systems (zumindest die Röntgenquelle) befestigt. Die Trommel ist auf Laufwagen gelagert, welche Tandemrollen umfassen, die um eine Achse parallel zur Drehachse der Trommel drehbar sind. Die Laufflächen der Rollen und der Laufwagen sind aus elastischem Material gefertigt und dämpfen somit eine Schwingungsübertragung zwischen dem Rahmen und der Trommel und nehmen zyklische Temperaturänderungen der Trommel auf. Das periphere, ringförmige Bauteil der Trommel wird durch ein gewalztes Strangpressteil aus einem leichten Material wie z. B. Aluminium bzw. einer Mg-Al-Legierung gebildet. Das Bauteil bildet ein gleichförmiges, kreisförmiges Band mit in Querrichtung vorspringenden Randlippen, die sich in Radialrichtung nach aussen erstrecken. Zusätzlich zu den Laufwagen weist die Vorrichtung mindestens drei Paare von elastischen, mit Reifen versehenen Rollen auf, die am Rahmen für eine Drehung entlang von radialen Linien angeordnet sind. Diese Rollen kontaktieren die Seitenkanten der Randlippen der Trommel und führen letztere somit in axialer Richtung. Der Antrieb der Trommel erfolgt durch einen Motor, der an eine der Tandemrollen gekoppelt ist.

Die US 7,010,081 B2 (Philips) zeigt einen CT-Scanner mit einer rotierenden Gantry und einer feststehenden Gantry, wobei die rotierende Gantry eine umlaufende Lagerschiene aufweist, welche zur axialen sowie radialen Lagerung zueinander geneigte Laufbahnen aufweist. An der feststehenden Gantry angeordnete konische Rollen wirken paarweise mit den Laufbahnen der Lagerschiene zusammen. Ein Antriebsmotor ist an eine der Rollen zum Antrieb der rotierenden Gantry gekoppelt. Die Rollenlager bestehen aus einem metallischen Kern, insbesondere aus Stahl, und einer reifenartigen Polymerbeschichtung, insbesondere aus Polyurethan, welche eine kissenartige Abfederung bei gleichzeitiger steifer Lagerung schafft.

Es hat sich gezeigt, dass bei derartigen Vorrichtungen die Drehbewegung der Gantry nicht genau steuerbar ist und dass unerwünschte Walkbewegungen der Gantry auftreten können.

Die US 5,109,397 (Analogic) zeigt einen Computertomographen mit einer ringförmigen Gantry. Diese ist an einem vertikalen Stützrahmen um eine horizontale Achse schwenkbar befestigt, wobei dieser Stützrahmen entlang von Schienen relativ zu einer Gerätebasis linear verschoben werden kann. Ein längenverstellbarer Hebel ist zwischen dem Stützrahmen und der Gantry befestigt und ermöglicht das Verschwenken der Gantry. Die Gantry umfasst einen äusseren ringförmigen Rahmen sowie einen drehbaren Innenteil, welcher über Wälzlager innen am Rahmen befestigt ist.

Diese Lösung ist konstruktiv aufwendig.

Die US 6,188,743 B1 (Analogic) betrifft einen Computertomographen mit einer ringförmigen Scheibe, deren umfangsseitige Kante gerillt ist, so dass die Scheibe als Riemenscheibe dienen kann, welche durch einen Antriebsmotor über einen gespannten Riemen antreibbar ist. Die Scheibe ist an einem stationären Rahmen gelagert, entweder im Bereich ihres Schwerpunktes über Distanzstücke oder umfangsseitig durch übliche Vier-DrahtLager. Die Scheibe kann aus einem leichten, steifen Material wie z. B. Aluminium oder einer Magnesium-Aluminium-Legierung gefertigt sein und wird vorzugsweise durch Präzisionsguss hergestellt und anschliessend gehärtet und endbearbeitet.

Übliche Drahtlager besitzen Lötstellen der Drähte, welche bei einer hohen Drehgeschwindigkeit der Scheibe zu Vibrationen führen. Diese Vibrationen verursachen ein Untergrundsignal in den Tomographieaufnahmen.

### Darstellung der Erfindung

Aufgabe der Erfindung ist es, ein dem eingangs genannten technischen Gebiet zugehörendes Trägersystem zu schaffen, welches kurze Untersuchungszeiten ermöglicht, aber trotzdem kostengünstig aufgebaut ist.

Die Lösung der Aufgabe ist durch die Merkmale des Anspruchs 1 definiert. Gemäss der Erfindung besteht die Trommel im Wesentlichen aus vergütetem Stahl und am Rahmen ist ein Laufrollensystem mit Laufrollen befestigt, mittels welchem die Trommel um die Rotationssymmetrieachse der Trommel drehbar ist. Die Laufrollen bestehen aus gehärtetem Stahl oder sind mindestens mit Laufflächen aus gehärtetem Stahl bestückt.

Durch die Herstellung der Trommel aus vergütetem Stahl und durch die Laufrollen, welche zumindest Laufflächen aus gehärtetem Stahl aufweisen, können erhöhte Drehgeschwindigkeiten der Trommel erreicht werden, ohne dass zugleich vermehrt Vibrationen der Trommel auftreten. Die so erreichten Drehgeschwindigkeiten ermöglichen kurze Untersuchungszeiten des Scannergerätes, da vibrationsbedingte Untergrundsignale vermieden werden. Ausserdem wird eine unvorteilhafte Geräuschentwicklung während der Untersuchung vermieden, welche den Komfort für die Nutzer des Scannergerätes verringern würde.

Besonders bevorzugt bestehen die Laufrollen aus Kugellagerstahl und sind durchgehärtet.

Die Lagerung der Trommel auf einem Rollensystem, welche erhöhte Drehgeschwindigkeiten der Trommel erlaubt, ist einfach und kostengünstig herzustellen.

Somit eignet sich das erfindungsgemässe Trägersystem für Scannergeräte besonders für Anwendungen, bei welchen kostengünstig eine kurze Untersuchungszeit erwünscht ist. Dies ist nebst der Computertomographie im medizinischen Bereich auch speziell in der Gepäckabfertigung an Flughäfen oder am Zoll der Fall.

Mit Vorteil ist die aus vergütetem Stahl hergestellte Trommel fugenlos hergestellt, insbesondere fugenlos gewalzt oder geschmiedet. Dadurch werden Vibrationen verhindert, welche entstehen würden, wenn eine der Laufrollen eine Fuge in der Trommel passiert. Dies erlaubt eine weitere Erhöhung der Drehgeschwindigkeit von der Trommel ohne eine unerwünschte Zunahme der Vibrationen.

Vorzugsweise verfügt die Trommel auf der Mantelfläche über eine Schiene, auf welcher die Laufrollen des Laufrollensystems laufen. Vorteilsweise ist diese Schiene zusammen mit der Trommel einstückig aus demselben Material gefertigt. Die Schiene weist zudem vorzugsweise induktionsgehärtete Laufflächen auf.

Bevorzugt lagert das Gewicht der Trommel auf mehreren Laufrollenpaaren, welche je auf einer Wippe montiert sind. Dabei sind sowohl die Drehachsen der Wippen als auch die Drehachsen der Laufrollen parallel zur rotationssymmetrischen Achse der Trommel orientiert. Die Drehachse der Wippen befindet sich jeweils bevorzugt auf der spiegelsymmetrischen Achse zwischen den beiden Laufrollen, welche senkrecht zur Verbindungslinie zwischen den beiden Laufrollen verläuft. Diese Lagerung hat den Vorteil, dass eine selbstregulierende, gleichmässige Verteilung der Gewichtslast der Trommel auf die an den Wippen montierten Laufrollen stattfindet. Zusätzlich hat diese Lagerung den Vorteil, dass kleine, temperaturbedingte Grössen- und Formänderungen der Trommel ausgeglichen werden. Da sich diese Lagerung der Trommel senkrecht zur rotationssymmetrischen Achse der Trommel anpasst ohne der Trommel dabei eine Translationsfreiheit senkrecht zur rotationssymmetrischen Achse der Trommel zu geben, werden auch Vibrationen unterdrückt, welche bei hoher Drehgeschwindigkeit der Trommel aufgrund einer Nichtübereinstimmung des Schwerpunktes der Trommel mit der Rotationsachse der Trommel entstehen können.

Alternativ zu dieser Lagerung ist auch eine Lagerung auf nur zwei Laufrollen oder eine Lagerung der Trommel auf mehreren exzentrisch gelagerten einzelnen Laufrollen denkbar.

Bevorzugt sind eine oder mehrere Laufrollen, auf welchen das Gewicht der Trommel nicht lagert, exzentrisch gelagert. Unter einer exzentrischen Lagerung wird im Folgenden eine exzentrische Lagerung der Rollenachsen verstanden, wobei der Exzenter bei der Montage einmal eingestellt wird und danach fixiert bleibt. Diese Laufrollen haben nur die Funktion, eine spielfreie Lagerung der Trommel zu gewährleisten. Da die Laufrollen entweder ganz aus gehärtetem Stahl hergestellt sind oder zumindest eine Lauffläche aus gehärtetem Stahl besitzen, ist hierfür eine exzentrische Lagerung der entsprechenden Laufrollen die beste Lösung.

Alternativ dazu können auch eine oder mehrere Laufrollen, welche aus weichem Material bestehen, verwendet werden. Solche Laufrollen passen sich der Grösse und Form der Trommel an, ohne dass sie dazu exzentrisch gelagert sein müssten.

Vorzugsweise wird die Trommel durch am Rahmen angebrachte Führungsrollen im Rahmen gehalten. Dazu laufen die Führungsrollen auf beiden Seiten der Schiene, wobei ihre Achsen radial zur Trommel ausgerichtet sind. Diese Führungsrollen sind ganz aus gehärtetem Stahl (insbesondere Kugellagerstahl) hergestellt oder mindestens mit einer Lauffläche aus gehärtetem Stahl bestückt.

Gegenüber einer ebenfalls denkbaren Anordnung, bei welcher die Führungsrollen auf den Stirnseiten der Trommel laufen, hat die Führungsrollenanordnung der Erfindung den Vorteil, dass die Instrumente und Komponenten des Scannergerätes auch an den Stirnseiten der Trommel angebracht werden können.

Gegenüber einer weiteren denkbaren Anordnung der Laufrollen und der Führungsrollen auf mehr als einer Schiene auf der Mantelfläche der Trommel besitzt diese Ausführung der Erfindung weiter den Vorteil, dass auf der Mantelfläche der Trommel Platz eingespart wird und somit das Scannergerät platzsparend gebaut werden kann.

Bevorzugt ist die Trommel ohne Spiel gelagert, indem die Führungsrollen auf mindestens einer Seite der Schiene exzentrisch gelagert sind. Dies ermöglicht eine spielfreie Führung der Trommel und damit einen ruhigen und gleichmässigen Lauf bei geringer mechanischer Belastung.

Vorzugsweise wird die Rotationsbewegung der Trommel durch einen Treibriemen angetrieben, welcher um die Trommel läuft. Dabei kann es sich um irgendeinen Treibriemen, beispielsweise um einen Keilriemen, einen Flachriemen oder einen Zahnriemen, handeln und der Treibriemen kann in einer Nut in der Mantelfläche der Trommel oder auch direkt auf der Mantelfläche der Trommel laufen. Bevorzugt weist die Mantelfläche einer Trommel, welche mit einer Schiene versehen ist, seitlich der Schiene eine integrierte Riemenscheibe auf, insbesondere ist die Mantelfläche in diesem Bereich entsprechend profiliert.

Der Vorteil des Antriebs über einen Treibriemen ist, dass ein guter Kraftübertrag auf die Trommel stattfindet. Alternative Antriebslösungen wie zum Beispiel der Antrieb über eine antreibende Laufrolle oder eine Antriebsrolle erfordern einen rutschfreien Kontakt zwischen der Schiene und der antreibenden Rolle. Ein solcher Antrieb ist nur über eine spezielle Lauffläche der antreibenden Rolle und/oder durch ein starkes Anpressen der antreibenden Rolle an die Trommel zu erreichen. Ersteres würde das Drehverhalten der Trommel negativ beeinflussen, während Letzteres einen hohen Energieverlust durch Reibung verursachen würde.

Bevorzugt ist der Motor für den Treibriemen am Rahmen befestigt. Dies ergibt den Vorteil, dass weder die Steuerung noch die Stromversorgung für den Motor vom Rahmen auf die Trommel geführt werden muss.

Bevorzugt wird der Treibriemen durch eine Spannrolle gespannt, welche am Rahmen angebracht ist. Diese Spannrolle hat den Vorteil, dass der Treibriemen auch im Betrieb des Trägersystems mit einer gewünschten Kraft gespannt werden kann und dass der Treibriemen im Reparaturfall auf einfache Art und Weise ersetzt werden kann.

Vorzugsweise ist ein Schleifring auf der Aussenseite der Trommel angebracht, durch welchen die benötigten elektrischen Kontakte zu den an der Trommel befestigten Instrumenten und Komponenten hergestellt werden, wobei eine am Rahmen befestigte Bürste die Kontakte zum Schleifring herstellt. Alternativ dazu können auch die benötigten elektrischen Kontakte an der Trommel und der Schleifring fest am Rahmen angebracht sein.

Vorzugsweise gewährleistet der Kontakt zum Schleifring auf der Aussenseite der Trommel die genaue Positionsbestimmung der Trommel.

Der Vorteil dieser Art der Positionsbestimmung ist, dass sie entgegen einer Bestimmung der Position des Antriebmotors direkt an der Trommel erfolgt und somit unmittelbar deren Position ermittelt.

Bevorzugt sind die Instrumente und Komponenten an Löchern in der Trommel befestigt.

Diese Art der Befestigung von Instrumenten und Komponenten weist den Vorteil auf, dass erfindungsgemäss eine Standardausführung des Trägersystems für verschiedene Scannergeräte verwendet werden kann und dass ein solches Scannergerät mit einfachen Mitteln mit weiteren oder neueren Instrumenten und Komponenten ausgebaut werden kann.

Ausserdem ist diese Art der Befestigung gewichtssparend, da keine schweren Zwischenstücke benötigt werden.

Eine alternative Möglichkeit zur Befestigung der Instrumente und Komponenten an der Trommel ist, dass eine Scheibe an der Trommel befestigt wird, auf welcher die Instrumente und Komponenten fest montiert sind.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine Schrägansicht eines erfindungsgemässen Trägersystems für ein Scannergerät,
- Fig. 2: eine Frontansicht des erfindungsgemässen Trägersystems, und
- Fig. 3: eine weitere Schrägansicht des erfindungsgemässen Trägersystems.
Grundsätzlich sind in den Figuren gleiche Teile mit gleichen Bezugszeichen versehen.

### Wege zur Ausführung der Erfindung

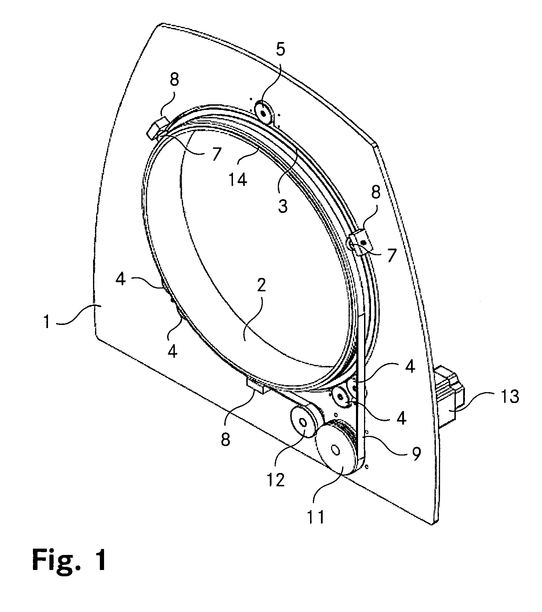

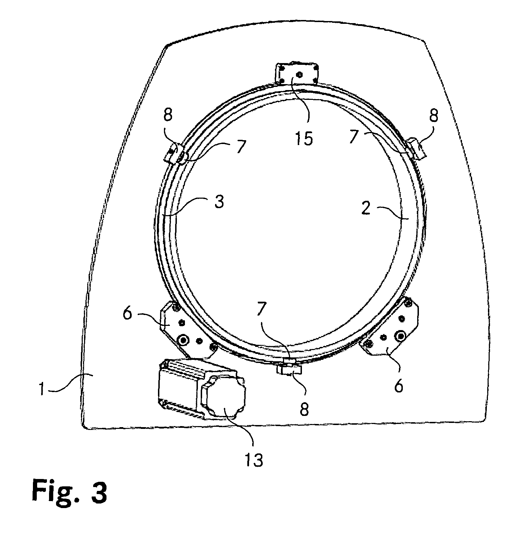

Die Figur 1 zeigt eine Schrägansicht eines erfindungsgemässen Trägersystems für Scannergeräte. Das gezeigte Trägersystem umfasst einen Rahmen 1, welcher aus einer Stahlplatte besteht und welcher aufrecht in einem Ständer (nicht gezeigt) befestigt ist. In diesem Rahmen 1 befindet sich eine Öffnung, in welcher eine im Wesentlichen hohlzylindrische Trommel 2 mit ihrer rotationssymmetrischen Achse senkrecht zur Ebene der Stahlplatte des Rahmens 1 Platz findet. Der Durchmesser dieser Trommel 2 ist etwa achtmal grösser als die Länge der Trommel 2. Die Trommel 2 ist aus vergütetem Stahl C45 (nach DIN EN 10027-1) fugenlos geschmiedet. Eine Schiene 3 mit rechteckigem Querschnitt, welche in der Mitte der Mantelfläche der Trommel 2 verläuft, umspannt die Trommel 2 ringförmig. Die Schiene 3 ist zusammen mit der Trommel 2 aus einem Stück geschmiedet. Die drei freien Flächen der Schiene sind induktionsgehärtet (58 (0/+4) HRC). Die Trommel 2 ist so im Rahmen 1 gelagert, dass die ringförmige Schiene 3 und der Rahmen 1 im Wesentlichen in einer Ebene liegen. Die ringförmige Schiene 3 lagert dabei auf der Aussenseite (d. h. der dem Rahmen 1 zugewandten Seite) an drei Stellen an unteren Laufrollen 4 und einer oberen Laufrolle 5. Diese unteren und obere Laufrollen 4, 5 sind entweder ganz aus durchgehärtetem (59 (0/+4) HRC) Kugellagerstahl (100Cr6 nach DIN EN 10027-1) gefertigt oder aber sie besitzen Laufflächen aus durchgehärtetem (59 (0/+4) HRC) Kugellagerstahl (100Cr6 nach DIN EN 10027-1). Die unteren Laufrollen 4 und die obere Laufrolle 5 sind am Rahmen 1 montiert. In einem Polarkoordinatensystem, bei welchem die Winkelkoordinate beim obersten Punkt in der Öffnung des Rahmens 1 bei 0 Grad beginnt und dann im Uhrzeigersinn in der Ebene des Rahmens 1 dreht, ist je ein Paar der unteren Laufrollen 4 bei 135° und bei 225° am Rahmen 1 montiert. Da der Rahmen 1 vertikal steht, lastet das Gewicht der Trommel 2 auf diesen beiden Paaren von unteren Laufrollen 4. Beide Paare der unteren Laufrollen 4 sind mit den Achsen der unteren Laufrollen 4 parallel zur rotationssymmetrischen Achse der Trommel 2 auf einer Wippe 6 in einer passenden Aussparung der Öffnung des Rahmens 1 montiert (siehe Figur 3; In der Ansicht der Figur 1 liegen diese Wippen 6 auf der Rückseite des Rahmens 1). Die Wippen 6 sind um eine Achse wippbar, welche parallel zur rotationssymmetrischen Achse der Trommel 2 liegt. Die Achse der Wippen 6 liegt in der spiegelsymmetrischen Ebene der Wippen 6, welche senkrecht zur Verbindungslinie zwischen den beiden Achsen der unteren Laufrollen 4 liegt. Die durch die Wippen 6 ermöglichte Wippbewegung sorgt dafür, dass das Gewicht der Trommel 2 gleichmässig auf alle unteren Laufrollen 4 verteilt ist.

Bei 0° der Winkelkoordinate des erwähnten Polarkoordinatensystems ist die obere Laufrolle 5 exzentrisch gelagert und klemmt die Trommel 2 zwischen sich und die unteren Laufrollen 4. Zusammen verhindern die unteren und oberen Laufrollen 4, 5 eine Verschiebung der Trommel 2 in der Ebene des Rahmens 1 und erlauben gleichzeitig eine Drehbewegung der Trommel 2 um ihre rotationssymmetrische Achse.

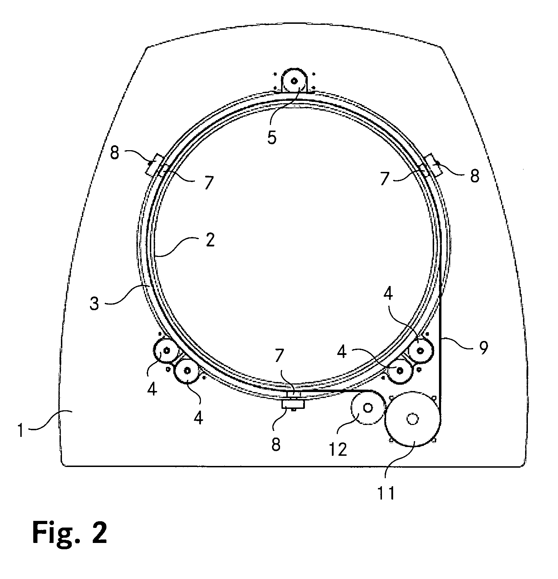

Damit die Schiene 3 der Trommel 2 und der Rahmen 1 in einer Ebene bleiben, sind am Rahmen 1 Führungsrollen 7 angebracht, welche aus durchgehärtetem (59 (0/+4) HRC) Kugellagerstahl (100Cr6 nach DIN EN 10027-1) bestehen oder welche zumindest Laufflächen aus durchgehärtetem Kugellagerstahl (100Cr6 nach DIN EN 10027-1) besitzen. Diese Führungsrollen 7 sind jeweils in Paaren angeordnet, wobei pro Paar die eine Führungsrolle 7 auf der einen Seite der Fläche des Rahmens 1 (und der Schiene 3) und die andere Führungsrolle 7 auf der anderen Seite des Rahmens 1 (und der Schiene 3) angebracht ist und auf der jeweiligen Seitenfläche der Schiene 3 läuft. Insgesamt sind drei Paar Führungsrollen 7 symmetrisch bei den Winkelkoordinaten 45°, 180° und 315° um die Trommel 2 angeordnet (siehe Figur 2). Die Achsen der Führungsrollen 7 zeigen radial von der rotations-symmetrischen Achse der Trommel 2 weg. Auf der von der rotationssymmetrischen Achse der Trommel 2 abgewandten Seite der Führungsrollen 7 werden die Achsen der Führungsrollen 7 von Führungsrollenhaltern 8 gehalten. Diese Führungsrollenhalter 8 sind fest am Rahmen 1 montiert. Mindestens auf der einen Seite des Rahmens 1 sind die Führungsrollen 7 exzentrisch gelagert, wodurch die Trommel 2 spielfrei im Rahmen 1 gelagert ist.

Auf der den Wippen 6 gegenüberliegenden Seite des Rahmens 1 ist ein Keilriemen 9 angeordnet, welcher in einer in der Trommel 2 eingearbeiteten Nut läuft. Der Keilriemen 9 wird von einer Antriebsrolle 11 angetrieben. Er umläuft die Trommel 2 und die Antriebsrolle 11 ohne sich zu überkreuzen. Der untere Teil des Keilriemens 9 wird gleich nach der Antriebsrolle 11 von einer Spannrolle 12 hochgedrückt und so gespannt. Sowohl die Antriebsrolle 11, als auch die Spannrolle 12 sind am Rahmen 1 montiert. Die Achse der Antriebsrolle führt durch den Rahmen 1 hindurch und wird auf der anderen Seite des Rahmens 1 von einem Motor 13 angetrieben.

Auf der Trommel 2 ist auf der vom Rahmen 1 abgewandten Seite des Keilriemens 9 ein Schleifring 14 an einer dafür vorgesehenen Schnittstelle angebracht, welcher über eine am Rahmen 1 befestigte Bürste (nicht gezeigt) die elektrischen Kontakte für die Instrumente und Komponenten, welche an der Trommel 2 befestigt sind (nicht gezeigt), herstellt. Dieser Kontakt zum Schleifring 14 ermöglicht zudem eine genaue Bestimmung der Position der Trommel 2. Entsprechende Schleifringsysteme sind bekannt und werden beispielsweise von der Firma Schleifring, Fürstenfeldbruck, Deutschland angeboten.

Die Figur 2 zeigt eine Frontansicht des erfindungsgemässen Trägersystems für Scannergeräte. Gut sichtbar ist hier die symmetrische Anordnung der unteren Laufrollen 4 bei den Winkelkoordinaten 135° und 225° und der oberen Laufrolle 5 bei der Winkelkoordinate 0° sowie der Führungsrollen 7 und der Führungsrollenhalter 8 bei den Winkelkoordinaten 45°, 180° und 315° um die Trommel 2 herum.

Die Figur 3 zeigt eine Schrägansicht des erfindungsgemässen Trägersystems für Scannergeräte. Im Unterschied zu Figur 1 und 2 zeigt diese Ansicht die Rückseite des Trägersystems.

Die beiden Wippen 6 sind am Rahmen 1 montiert und halten je zwei untere Laufrollen 4. (Die unteren Laufrollen 4 sind nur von der anderen Seite her sichtbar; siehe Figur 1 und 2.)

Ein Gehäuse 15 ist am Rahmen 1 befestigt und beherbergt die exzentrisch gelagerte obere Laufrolle 5. (Die obere Laufrolle 5 ist nur von der anderen Seite her sichtbar; siehe Figur 1 und 2.)

Auf der hier gezeigten Seite der Trommel 2 befindet sich ein Lochmuster (nicht gezeigt), welches die Befestigung der Instrumente und Komponente (nicht gezeigt) an der Trommel 2 ermöglicht.

Die Erfindung ist nicht auf das dargestellte Ausführungsbeispiel beschränkt. Abhängig von der konkreten Anwendung kann beispielsweise der Rand der Trommel 2 zwischen den an der Trommel 2 befestigten Instrumenten und Komponenten Aussparungen aufweisen, wodurch die Gesamtmasse der Trommel 2 reduziert wird. Dies erlaubt die Verwendung eines weniger stark ausgelegten Motors 13 und eines entsprechend weniger starken Keilriemens.

Des Weiteren kann beispielsweise der Keilriemen 9 durch einen Zahnriemen oder andere Arten von Treibriemen ersetzt werden.

Abhängig von der konkreten Anwendung kann beispielsweise auch die Anordnung des Treibriemens, des Schleifrings 14 sowie der Löcher zur Befestigung von Instrumenten und Komponenten auf der Trommel 2 variiert werden. Dabei können sowohl Treibriemen, Schleifring 14 als auch Löcher auf der einen oder anderen Seite des Rahmens 1 auf der Trommel 2 platziert werden.

Je nach Anwendung des Trägersystems kann die Anordnung der Laufrollen 4, 5 geändert werden. Zudem kann mehr als eine Laufrolle 5 verwendet werden. Dementsprechend können je nach Bedürfnis die Positionen der Führungsrollen 7 variiert werden.

Zusammenfassend ist festzustellen, dass durch die Erfindung ein Trägersystem für Scannergeräte geschaffen wird, welches kurze Untersuchungszeiten ermöglicht, aber trotzdem kostengünstig aufgebaut ist.

## Patentansprüche

1. Trägersystem für ein Scannergerät, welches einen Rahmen (1) und eine Trommel (2) umfasst, wobei eine Lagerung der Trommel (2) am Rahmen (1) eine Rotation der Trommel (2) um deren rotationssymmetrische Achse erlaubt und wobei an der Trommel (2) ein Scannersystem befestigbar ist, **dadurch gekennzeichnet, dass** die Trommel (2) im Wesentlichen aus vergütetem Stahl besteht und dass am Rahmen (1) ein Laufrollensystem mit Laufrollen (4, 5) befestigt ist, mittels welchem die Trommel (2) um die Rotationssymmetrieachse der Trommel (2) drehbar ist, wobei die Laufrollen (4, 5) aus gehärtetem Stahl bestehen oder mindestens mit Laufflächen aus gehärtetem Stahl bestückt sind.

2. Trägersystem nach Anspruch 1, **dadurch gekennzeichnet, dass** die Trommel (2) fugenlos hergestellt, insbesondere geschmiedet oder gewalzt, ist.

3. Trägersystem nach Anspruch 1 oder 2, **dadurch gekennzeichnet, dass** die Trommel (2) auf der Mantelfläche über eine Schiene (3) verfügt, auf welcher die Laufrollen (4, 5) des Laufrollensystems laufen.

4. Trägersystem nach einem der Ansprüche 1 - 3, **dadurch gekennzeichnet, dass** das Gewicht der Trommel (2) auf mehreren Laufrollenpaaren (4) lagert, welche je auf einer Wippe (6) montiert sind.

5. Trägersystem nach einem der Ansprüche 1 - 4, **dadurch gekennzeichnet, dass** eine oder mehrere weitere Laufrollen (5), auf welchen das Gewicht der Trommel (2) nicht lagert, exzentrisch gelagert sind.

6. Trägersystem nach einem der Ansprüche 1 - 5, **dadurch gekennzeichnet, dass** die Trommel (2) durch am Rahmen (1) angebrachte Führungsrollen (7) im Rahmen (1) gehalten wird, indem die Führungsrollen (7) auf beiden Seiten der Schiene (3) laufen und indem die Achsen der Führungsrollen (7) radial zur Trommel (2) ausgerichtet sind, wobei die Führungsrollen (7) ganz aus gehärtetem Stahl gefertigt sind oder mindestens mit einer Lauffläche aus gehärtetem Stahl bestückt sind.

7. Trägersystem nach Anspruch 6, **dadurch gekennzeichnet, dass** die Trommel (2) ohne Spiel gelagert ist, indem auf mindestens einer Seite der Schiene (3) die Führungsrollen (7) exzentrisch gelagert sind.

8. Trägersystem nach einem der Ansprüche 1 - 7, **dadurch gekennzeichnet, dass** die Rotationsbewegung der Trommel (2) über einen um die Trommel (2) laufenden Treibriemen (9) angetrieben ist.

9. Trägersystem nach einem der Ansprüche 1 - 8, **dadurch gekennzeichnet, dass** der Motor (13) für den Treibriemen (9) am Rahmen (1) befestigt ist.

10. Trägersystem nach einem der Ansprüche 1 - 9, **dadurch gekennzeichnet, dass** der Treibriemen (9) durch eine Spannrolle (12) gespannt wird.

11. Trägersystem nach einem der Ansprüche 1 - 10, **dadurch gekennzeichnet, dass** ein Schleifring (14) auf der Aussenseite der Trommel (2) die benötigten elektrischen Kontakte zu den Instrumenten, welche an der Trommel (2) befestigt sind, herstellt.

12. Trägersystem nach einem der Ansprüche 1 - 11, **dadurch gekennzeichnet, dass** durch einen Kontakt zum Schleifring (14) auf der Aussenseite der Trommel (2) die genaue Positionsbestimmung der Trommel (2) gewährleistet ist.

13. Trägersystem nach einem der Ansprüche 1 - 12, **dadurch gekennzeichnet, dass** die Strahlungsquelle und der oder die Detektoren an Löchern in der Trommel (2) befestigt sind.
